# EUROPEAN PATENT APPLICATION

(11) **EP 1 955 655 A1**
(43) Date of publication of application: **13.08.2008**
(21) Application number: 06823511.8
(22) Date of filing: 22.11.2006
(51) Int. Cl.: A61B 8/00, G01N 29/28

(54) **GEL FOR ULTRASONIC PHANTOM**

(30) Priority: 29.11.2005 JP 2005343909
(71) Applicant: TAKIRON CO., LTD., Osaka-shi, Osaka 541-0052 (JP)
(72) Inventor: SHIKINAMI, Yasuo c/o TAKIRON CO., LTD., Chuo-ku, Osaka-shi 541-0052 (JP); TSUTA, Kaoru c/o TAKIRON CO., LTD., Chuo-ku, Osaka-shi 541-0052 (JP)
(74) Representative: HOFFMANN EITLE
(86) International application number: PCT/JP2006/323354
(87) International publication number: WO 2007/063762

(57) **Abstract**

A gel for an ultrasonic phantom is provided which has satisfactory ultrasonic characteristics, does not fluctuate in ultrasonic characteristics over long, and is free from germ multiplication, alteration, putrefaction, etc.

The gel is a gel for ultrasonic phantoms which comprises: a segmented polyurethane gel having alkylene oxide segments which are mostly or wholly liquid at ordinary temperature; and a nonvolatile organic gel-swelling medium not participating in a urethane-forming reaction, the medium being contained in the segmented polyurethane gel. Due to the nonvolatile organic gel-swelling medium, the liquid segments and the like are kept in an extended state and the distance between the segments is increased. The gel hence has satisfactory ultrasonic characteristics concerning ultrasonic attenuation rate, propagation velocity, etc. The gel does not change in swelling-medium content with time and hence retains the satisfactory ultrasonic characteristics over long. Furthermore, the gel is free fromgermmultiplication, alteration, putrefaction, etc. because of the organic gel-swelling medium contained therein.

## Description

### TECHNICAL FIELD

The present invention relates to an ultrasonic phantom (living-tissue model) gel suitable for purposes such as researches on medical ultrasonic waves for ultrasonic examinations of regions of the body, calibration of ultrasonic diagnostic apparatus, technical training of doctors and technicians, etc.

### BACKGROUND ART

Hydrogels for gel materials for phantoms have been investigated, such as a gel containing agar or gelatin as a base material, a gel containing glucomannan, which is contained in *konjak,* as a base material (patent document 1), a gel containing an acidic-water-containing poly(vinyl alcohol) as a base material (patent document 2), a gel containing a water-swelling urethane resin as a base material (patent document 3), and a gel containing a water-containing water-absorbing resin as a base material (patent document 4). Use of part of these hydrogels for researches on medical ultrasonic waves, calibration of ultrasonic diagnostic apparatus, technical training of doctors and technicians, etc. is being investigated. However, those conventional gels are unsatisfactory.

In general, an ultrasonic phantom for practical use is required to have the following properties. Namely, the phantom is required to have such ultrasonic characteristics, for example, that it has an ultrasonic propagation velocity which is as close as possible to that of human tissues and that the phantom has an ultrasonic attenuation rate which is intrinsically lower than that of human tissues and which can be regulated with an additive so as to be close to the attenuation rate of human tissues to be examined. The phantom is further required to retain such ultrasonic characteristics over a prolonged storage period without changing in the characteristics. In addition, the gel material as a phantom is required to be less apt to alter with time.

However, the gel-state ultrasonic phantom made of agar or gelatin containing water in a high proportion and the gel-state ultrasonic phantom based on glucomannan disclosed in patent document 1 have had the following drawbacks although satisfactory in those ultrasonic characteristics. These phantoms have a problem concerning germ multiplication, alteration, and putrefaction because they are hydrogels (hydrous gels), and there also has been a problem that these phantoms have low mechanical strength and hence readily break.

On the other hand, the ultrasonic phantom of patent document 2, which is made of an acidic-water-containing poly(vinyl alcohol) gel, the ultrasonic phantom of patent document 3, which is made of a water-containing swelling urethane gel, and the ultrasonic phantom of patent document 4, which employs another water-absorbing resin as a base material, each have a problem that since the phantom contains water in a high proportion, the ultrasonic characteristics decrease or fluctuate as the water flies off into the air and the water content changes with time. In addition, the unavoidable problem concerning germ multiplication remains unsolved. Ultrasonic phantoms made of a hydrogel, which contains water, inevitably have that problem concerning fluctuations in ultrasonic characteristics.
Patent Document 1: JP-A-2002-360572
Patent Document 2: JP-A-11-155856
Patent Document 3: JP-A-11-262487
Patent Document 4: JP-A-10-295692

### DISCLOSURE OF THE INVENTION

### PROBLEMS THAT THE INVENTION IS TO SOLVE

The invention has been achieved in order to overcome the problems described above. An object of the invention is to provide a gel which is for an ultrasonic phantom and which has sufficient ultrasonic characteristics, does not fluctuate in ultrasonic characteristics over long, and is free from germ multiplication, alteration, putrefaction, etc.

### MEANS FOR SOLVING THE PROBLEMS

In order to accomplish that obj ect, the invention provides a gel for an ultrasonic phantom, characterized by comprising: a segmented polyurethane gel having alkylene oxide segments which are mostly or wholly liquid at ordinary temperature; and a nonvolatile organic gel-swelling medium which does not participate in a urethane-forming reaction and has an ultrasonic propagation velocity close to that of water and a low attenuation rate, the medium being contained in the segmented polyurethane gel.

In the gel for an ultrasonic phantom of the invention, it is preferred that the alkylene oxide segments each should be a copolymer of ethylene oxide and propylene oxide, and that the molar ratio of ethylene oxide to propylene oxide in the copolymers should be from 20:80 to 80:20.

It is also preferred that in the gel for an ultrasonic phantom of the invention, the nonvolatile organic gel-swelling medium should be contained in the segmented polyurethane gel in an amount of 20-80% by weight. This organic gel-swelling medium preferably comprises any one member selected from or a mixture of any two or more members selected from propylene carbonate, ethylene carbonate, N-methyl-2-pyrrolidone, diethylene glycol dimethyl ether, diethylene glycol diethyl ether, triethylene glycol dimethyl ether, triethylene glycol diethyl ether, tetraethylene glycol dimethyl ether, tetraethylene glycol diethyl ether, diethylene glycol dipropyl ether, diethylene glycol dibutyl ether, polypropylene glycol dimethyl ether, and polypropylene glycol diethyl ether. The segmented polyurethane gel suitably contains an attenuation rate regulator for regulating ultrasonic attenuation rate. The attenuation rate regulator to be used preferably is one which comprises crosslinked or uncrosslinked beads of poly (methyl methacrylate), polystyrene, expanded polystyrene, or an acrylic or comprises beads of polyethylene, graphite, glass, or solid paraffin wax, and which has an average particle diameter of 1-100 µm.
In the invention, "% by weight" has the same meaning as "% by mass", and "parts by weight" has the same meaning as "parts by mass".

### ADVANTAGES OF THE INVENTION

For obtaining a gel having satisfactory ultrasonic characteristics for an ultrasonic phantom, it is desirable that most or all of the alkylene oxide segments of the segmented polyurethane gel shouldhave flowability at ordinary temperature and that the molecular chains constituting the segments be kept in a moderately extended state to increase the distance between the segments and keep the network chain structure in an expanded state as compared with the state before the addition of the swelling agent. In case where the segments are solid and rigid and have no flowability or where the segments have considerably bent and shrunk and are in a rounded state, this gel shows enhanced absorption of ultrasonic vibration energy and sound waves are not propagated with low resistance. Because of this, the attenuation of ultrasonic propagation in this segmented polyurethane gel is higher than that in tissues of an internal organ of the living body which contain 60-70% water. This may arouse a trouble that to add an attenuation rate regulator to regulate the attenuation is meaningless. However, in the gel for an ultrasonic phantom of the invention, the alkylene oxide segments are mostly or wholly liquid at ordinary temperature and are kept in a moderately extended state by the nonvolatile organic gel-swelling medium contained in the gel. Because of this, the liquid segments readily vibrate, with exceedingly low resistance, synchronously with ultrasonic vibration. This gel hence shows exceedingly low energy absorption, and attenuation is inhibited to the highest degree. Since the swelling medium intrinsically has the same ultrasonic characteristics as water, it is present among the segmented-polyurethane network chains for forming and maintaining a gel. As a result, satisfactory ultrasonic characteristics are obtained which are equal to or higher than those of target human soft tissues and are equal to those of blood and body fluids. Consequently, the gel of the invention can be constituted so as to have a lower ultrasonic attenuation rate than human internal-organ (soft) tissues and this attenuation rate can be moderately heightened by addition of an attenuation rate regulator to a value close to the ultrasonic attenuation rate of target human internal-organ tissues, as will be shown by experimental data given later. This gel for a phantom has an ultrasonic propagation velocity slightly lower than that of human internal-organ tissues as will be shown by experimental data given later. However, this propagation velocity is within a range in which the velocity can be set so as to be close to the ultrasonic propagation velocity of human internal-organ tissues by regulating an ultrasonic diagnosis apparatus or the like. The ultrasonic propagation velocity of the gel hence causes no problem in practical use.

The swelling medium contained in the segmented polyurethane gel in the invention is an organic gel-swelling medium not participating in a urethane-forming reaction. There is hence no fear that the urethane-forming reaction for gel formation may be inhibited or the polyurethane gel may be altered by the swelling medium. Furthermore, since this gel-swelling medium is an organic one and has a poor affinity for water, the gel is free from germ multiplication. Moreover, since the gel is a polyurethane gel, it is free from problems such as alteration and putrefaction. In addition, since this organic gel-swelling medium is nonvolatile, the content of the organic gel-swelling medium in the gel for an ultrasonic phantom of the invention is kept constant and does not change with time. The gel can hence retain the satisfactory ultrasonic characteristics over long (permanently).

### BEST MODE FOR CARRYING OUT THE INVENTION

Specific embodiments of the invention will be described below in detail.

The gel for an ultrasonic phantom of the invention comprises a segmented polyurethane gel having alkylene oxide segments which are mostly or wholly liquid at ordinary temperature and a nonvolatile organic gel-swelling medium which does not participate in a urethane-forming reaction and is contained in the segmented polyurethane gel. This segmented polyurethane gel is a one-component segmented polyurethane gel of the so-called three-dimensional interpenetrated network type obtained, for example, by using one of or a mixture of two or more of polyol ingredients represented by the following structural formula A to D and one of or a mixture of two or more of polyisocyanate ingredients represented by the following structural formula E to I and combining the -OH groups of the former with the -NCO groups of the latter through urethane bonds.

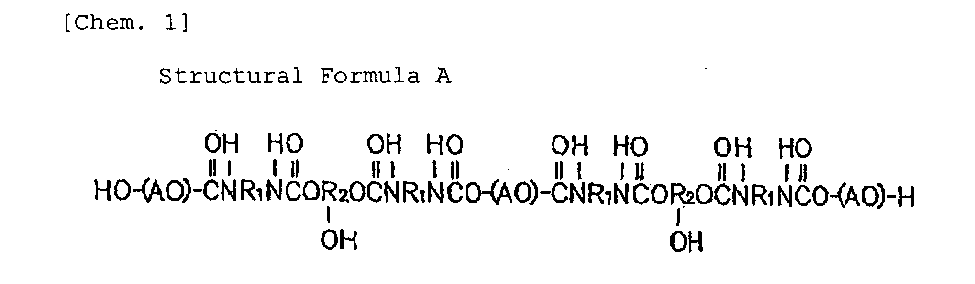

In the formula, R₁ and R₂ each is any of an alkyl compound, alicyclic compound, and aromatic compound, and AO is an alkylene oxide segment.

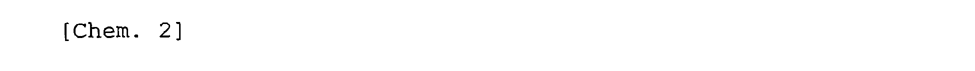

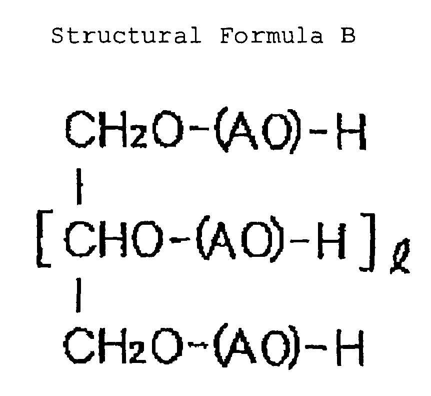

In the formula, AO is an alkylene oxide segment, and 1 is an integer of 1 or 4.

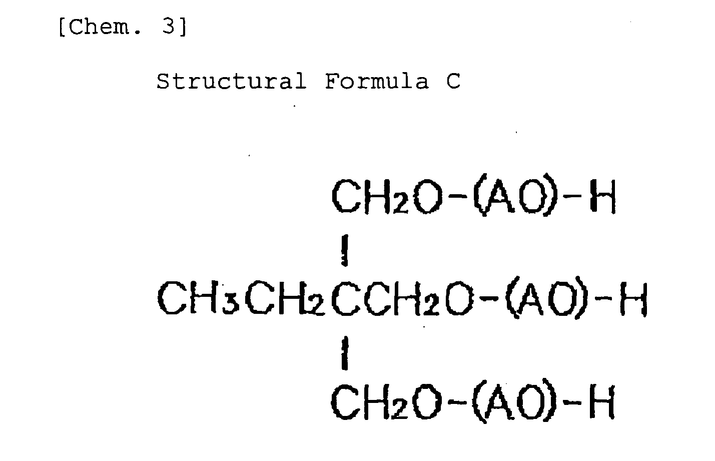

In the formula, AO is an alkylene oxide segment.

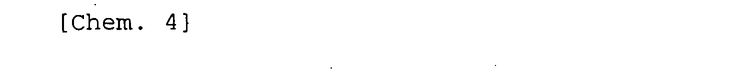

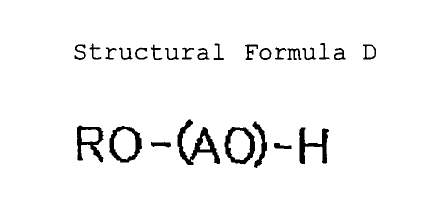

In the formula, AO is an alkylene oxide segment, and R is a hydrogen atom or any of an alkyl compound, alicyclic compound, and aromatic compound.

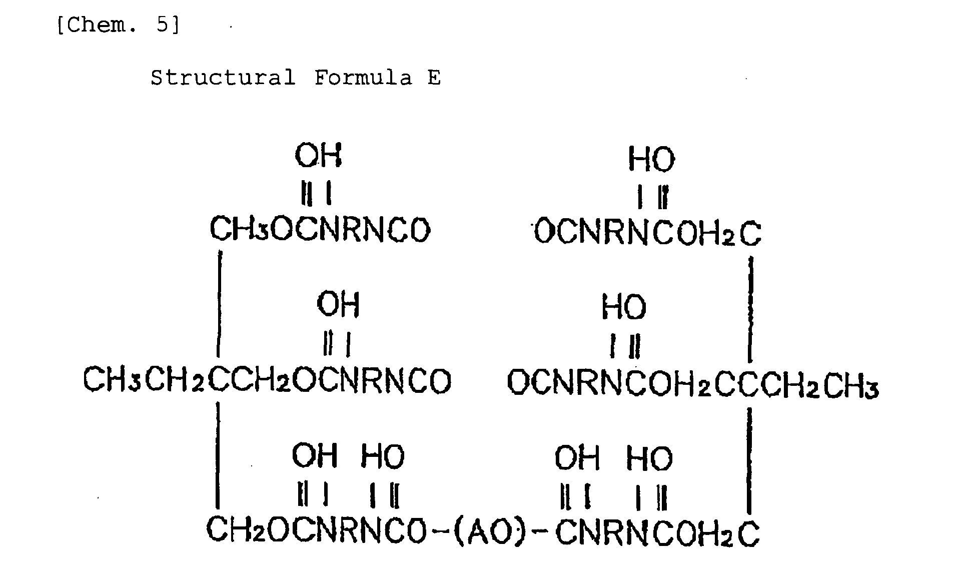

In the formula, R is any of an alkyl group, alicyclic compound, and aromatic compound, and AO is an alkylene oxide segment.

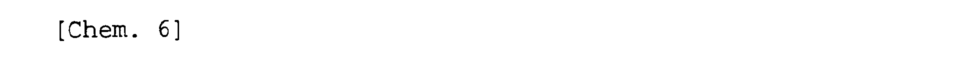

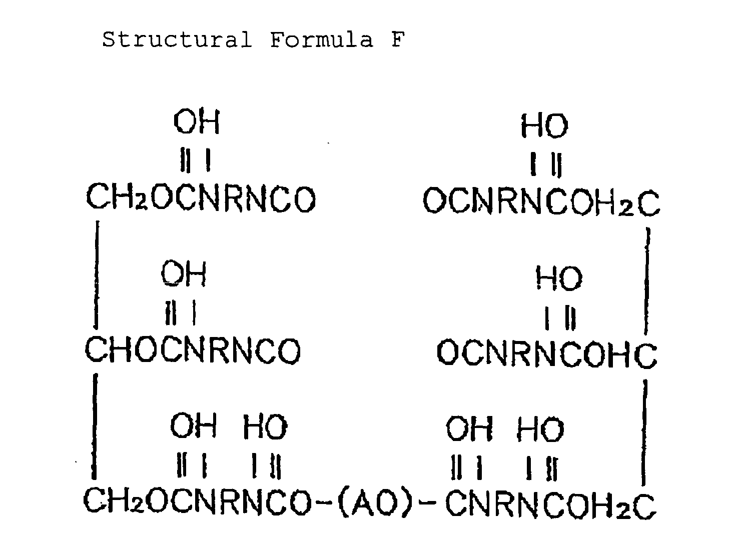

In the formula, R is any of an alkyl group, alicyclic compound, and aromatic compound, and AO is an alkylene oxide segment.

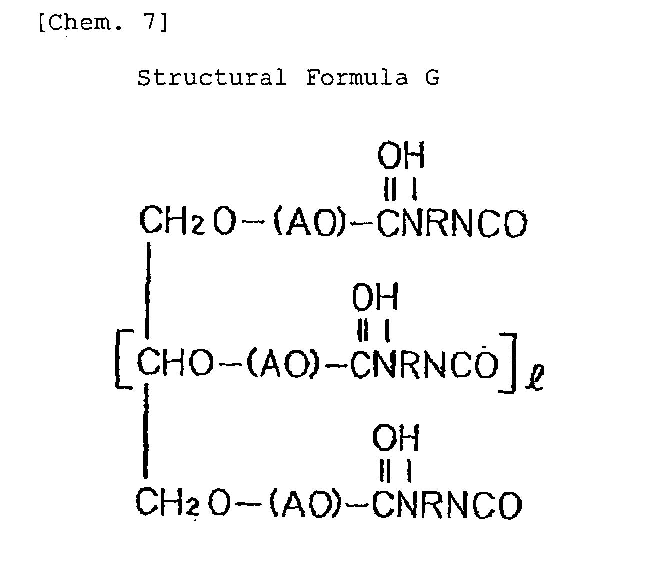

In the formula, R is any of an alkyl group, alicyclic compound, and aromatic compound, AO is an alkylene oxide segment, and 1 is an integer of 1 or 4.

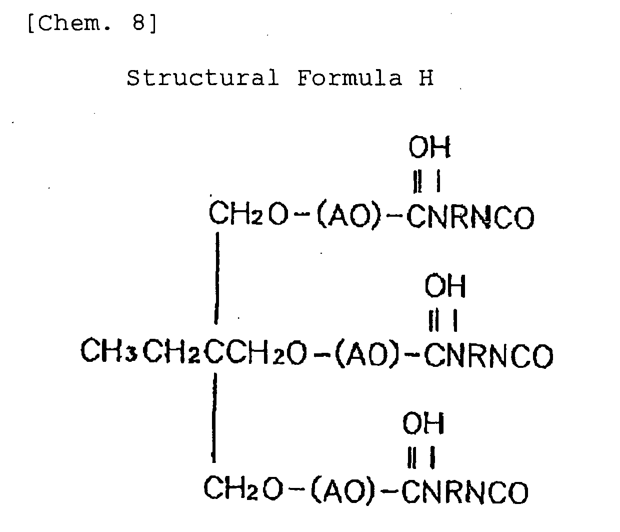

In the formula, R is any of an alkyl group, alicyclic compound, and aromatic compound, and AO is an alkylene oxide segment.

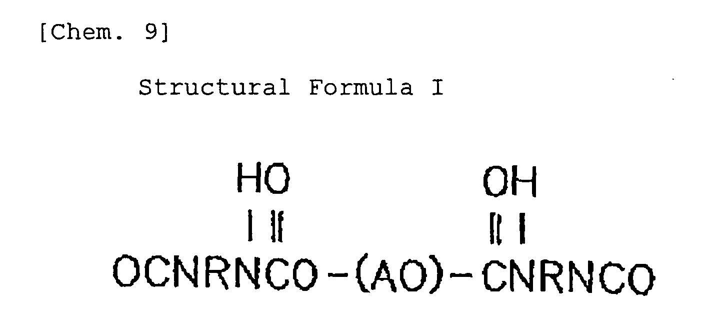

In the formula, R is any of an alkyl group, alicyclic compound, and aromatic compound, and AO is an alkylene oxide segment.

The prepolymers shown above as polyol ingredients represented by structural formula A to D are explained below. Structural formula A shows a polyurethane polyol prepolymer which is a product of the reaction of a polyether polyol with a diisocyanate and in which the two end components are polyether polyol moieties terminated by an -OH group. The diisocyanate compound used here is the same as that in the polyurethane polyisocyanate prepolymer which will be described later. For example, the following may be used at will: phenylene diisocyanate, 2,4-toluylene diisocyanate (TDI), 4,4'-diphenylmethane diisocyanate (MDI), naphthalene 1,5-diisocyanate, hexamethylene diisocyanate (HMDI), tetramethylene diisocyanate (TMDI), lysine diisocyanate, xylylene diisocyanate (XDI), hydrogenated TDI, hydrogenated MDI, dicyclohexyldimethylmethane p,p'-diisocyanate, diethyl fumarate diisocyanate, isophorone diisocyanate (IPDI), and the like.

Structural formula B shows a prepolymer formed by causing a polyether polyol to add to glycerol (1=1) or sorbitol (1=4), while structural formula C shows a prepolymer formed by causing a polyether to add to trimethylol propane. Likewise, use can be made of an adduct of a polyether polyol with a polyhydric alcohol such as, e.g., 1, 2, 6-hexanetriol of structural formula J, trimethylolethane of structural formula K, pentaerythritol of structural formula L, a polyglycerol of structural formula M (n=2-30), or a partial ester thereof.

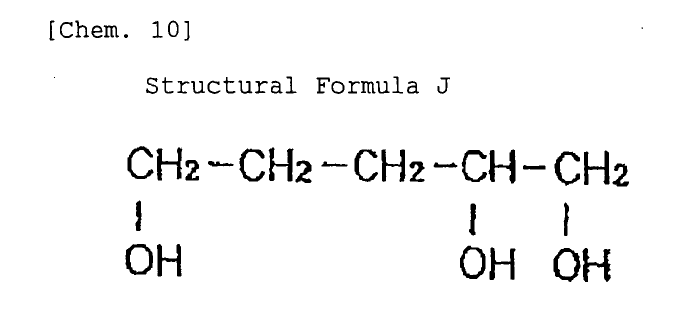

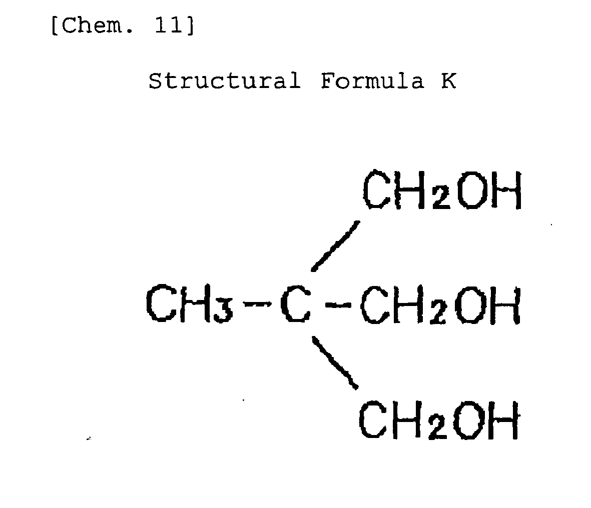

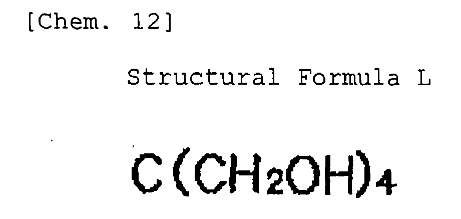

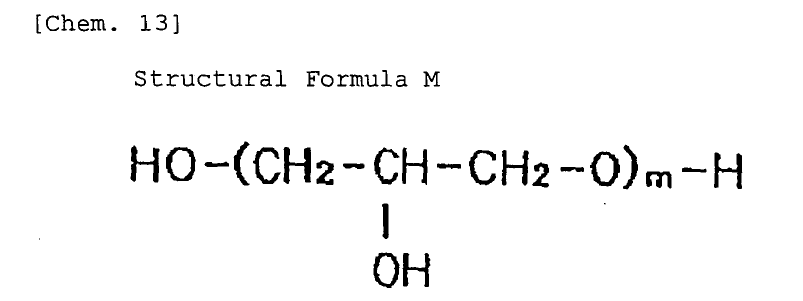

Structural formula D shows a polyether polyol having an alkylene oxide (AO) segment. There are cases where both ends each are an -OH group and cases where one end has been blocked with an alkyl group, aromatic group, etc. Commercial products thereof are easily available.

The polyisocyanate prepolymers represented by structural formula E to I are then explained. Structural formula E shows a prepolymer formed by reacting trimethylolpropane with a diisocyanate to obtain a triisocyanate and dimerizing the triisocyanate by combining two molecules thereof with one AO molecule. This prepolymer is a tetraisocyanate, which is tetrafunctional. A prepolymer obtained in the same manner except that glycerol is used in place of the trimethylolpropane is the compound of structural formula F. In producing a tetraisocyanate of this kind, the triisocyanate is apt to be dimerized with two or three AO molecules. It is therefore necessary to delicately control the reaction.

Structural formula G shows a prepolymer formed by reacting a diisocyanate with a polyol represented by structural formula B. Structural formula H likewise shows a prepolymer formed by reacting a diisocyanate with a polyol represented by structural formula C; this prepolymer is trifunctional. Structural formula I shows a product of the reaction of a polyether polyol with a diisocyanate; this prepolymer is bifunctional.

The alkylene oxide segments represented by AO in structural formula A to I must be segments which are mostly or wholly liquid at ordinary temperature, from the standpoint of obtaining a segmented polyurethane gel having satisfactory ultrasonic characteristics. Specifically, the alkylene oxide segments eachmaybe any of an ethylene oxide (EO) chain, propylene oxide (PO) chain, ethylene oxide/propylene oxide copolymer (EO-PO copolymer), butylene oxide (BO) chain, and BO-PO copolymer. Of these, an EO-PO copolymer is especially preferred. This is because EO and PO have a low attenuation rate and, hence, the AO segments constituted of EO-PO copolymers heighten the degree of freedom of composition investigations in producing phantoms having various properties.

The EO-PO copolymers may be any of, for example, block copolymers represented by the following structural formula N, alternating copolymers such as -(PO-EO-EO-PO)ₘ-, -(PO-PO-EO)ₘ-, -(EO-EO-PO)ₘ-, and -(EO-PO-EO-PO)ₘ- (m is a positive integer of 1 or larger), and random copolymers of EO and PO. However, random copolymers of EO and PO are especially preferred. This is because when the AO segments are random copolymers of EO and PO, the introduction of EO, which has a lower attenuation rate, into the segments in a high proportion prevents crystallization. Namely, this case is advantageous for maintaining the ultrasonic performance of the phantom in a low region.

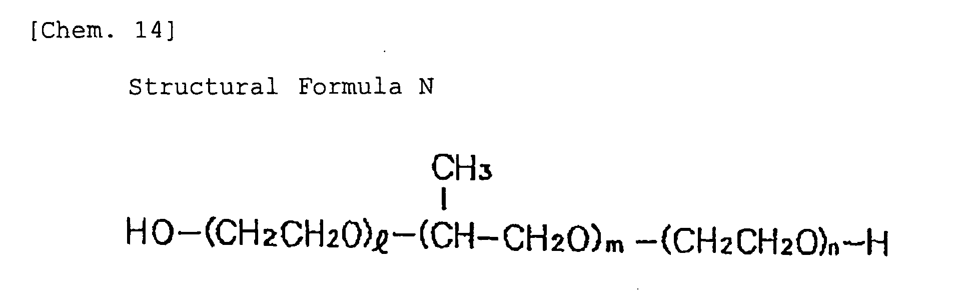

The AO segments each constituted of an EO chain, PO chain, EO-PO copolymer, BO chain, or BO-PO copolymer must be mostly or wholly liquid at ordinary temperature from the standpoint of obtaining a segmented polyurethane gel having satisfactory ultrasonic characteristics. There is hence an upper limit on the molecular weight thereof. Even when the AO segments are liquid, too low molecular weights thereof result in a reduced distance between crosslinking sites and an increased crosslink density and this gives a polyurethane gel having an increased ultrasonic attenuation rate. Because of this, there also is a lower limit on the molecular weight. The molecular weight (weight-average molecular weight MW; the same applies hereinafter) of the EO chain is desirably 150-1,000, preferably 300-800. In the case of the PO chain, it is liquid even when it has a molecular weight of tens of thousands and the range of usable molecular weights is wide. However, the lower the proportion of terminal groups, the lower the probability of reaction. In addition, too long segments give a polyurethane gel having enhanced flowability and poor shape retentivity. Consequently, the molecular weight thereof is preferably in the range of about from 200 to several thousand.

On the other hand, in the case of the EO-PO copolymer, the flowability (softness) and other properties thereof are influenced by the EO-PO molar ratio and the arrangement of the units. For example, an EO-PO block copolymer having a high PO molar proportion is liquid even when having a high molecular weight, while an EO-PO block copolymer having a low PO molar proportion is liquid when the EO has a low molecular weight. However, too high molecular weights of the PO chain arouse problems concerning shape retentivity, etc., and too low molecular weights of the copolymer result in an increased crosslink density to heighten the ultrasonic attenuation rate of the segmented polyurethane gel, as stated above. Because of this, use may be made of an EO-PO block copolymer, EO-PO alternating copolymer, or EO-PO random copolymer which has a molecular weight of 60-8,000, preferably 800-6,000, and is liquid at ordinary temperature.

The BO chain becomes solid as the degree of polymerization thereof increases. Consequently, the BO chain which canbe usedhas amolecular weight in the range of 200-1, 000, preferably 400-800. In the case of the BO-PO copolymer, it is liquid even when having a high molecular weight, so long as the PO molar proportion is high. Consequently, the BO-PO copolymer which can be used has a molecular weight in the range of 800-6,000, preferably 900-3,000.

The EO-PO copolymer constituting the AO segments preferably is one in which the EO-PO molar ratio is from 20:80 to 80:20. In case where the molar proportion of EO exceeds 80, this EO-PO copolymer shows enhanced crystallization and hence gives a segmented polyurethane gel having a heightened attenuation rate. On the other hand, in case where the proportion of PO exceeds 80 and is excessively high, this EO-PO copolymer has enhanced flowability to give a segmented polyurethane gel which has reduced shape retentivity, readily breaks, and has a heightened attenuation rate. The EO-PO molar ratio is more preferably from 50:50 to 70:30.

Also in the case where the segmented polyurethane gel has an EO chain segment and a PO chain segment (e.g., in the case where a polyol ingredient and a polyisocyanate ingredient either of which has an EO chain segment and the other of which has a PO chain segment are subjected to urethane-forming reaction), the EO/PO molar ratio is preferably from 20:80 to 80:20 as in the case described above.

Polyethylene glycol (PEG) may be used as a compound for forming an EO chain, and polypropylene glycol (PPG) may be used as a compound for forming a PO chain. A PEG-PPG block copolymer or random copolymer may be used as a compound constituting an EO-PO copolymer, and polybutylene glycol (PBG) may be used as a compound for forming a BO chain. Furthermore, a PBG/PPG copolymer may be used as a compound constituting a BO/PO copolymer.

The segmented polyurethane gel having satisfactory ultrasonic characteristics must have a bulky molecularstructure which is a three-dimensional network chain structure in which the AO segments and polymer chains derived from the polyol ingredient and polyisocyanate ingredient are in the state of being moderately extended by the organic gel-swelling medium contained and the distance between the AO segments or polymer chains has been increased. For obtaining a segmentd polyurethane gel having a bulky molecular structure which is such a three-dimensional network chain structure, it is preferred that either of the polyol ingredient and polyisocyanate ingredient to be subj ected to a urethane-forming reaction should be bifunctional and the other be trifunctional or tetrafunctional. In case where each of the polyol ingredient and polyisocyanate ingredient has a functionality of 3 or higher, the resultant polyurethane gel may have too high a network chain density and there is a possibility that the ultrasonic attenuation rate thereof might exceed that of tissues of the living body.

The ratio in which a polyol ingredient is reacted with a polyisocyanate ingredient can be controlled by regulating the proportions of terminal functional groups, i.e., the value of OH/NCO. In case where -NCO remains unreacted, an after-reaction occurs. The value of OH/NCO must hence be 1 or larger. When 1.5≤OH/NCO≤3.5, a polyurethane gel having satisfactory ultrasonic characteristics is obtained. In case where OH/NCO exceeds 3.5, the reaction yields a mass of bulky molecules which include a larger proportion of linear segments having a terminal OH group and moving freely. In this case, there is a possibility that the resultant polyurethane gel might have reduced ultrasonic characteristics because the linear segments adversely influence ultrasonic attenuation rate and propagation velocity.

The ranges of the molecular weights of the polyol ingredient and polyisocyanate ingredient vary widely depending on the kinds of the AO and isocyanate, molecular shapes thereof, whether each AO segment is a homopolymer or copolymer, etc. However, in case where the molecular weights thereof are too high, it is difficult for an organic gel-swelling medium to sufficiently extend and stretch the polymer chains formed from the polyol ingredient and polyisocyanate ingredient having AO segments. This results in reduced ultrasonic characteristics. In case where the molecular weights of the polyol ingredient and the polyisocyanate ingredient are too low, the resultant polyurethane gel has too high a crosslink density. Namely, this gel also has reduced ultrasonic characteristics. Consequently, the molecular weights of the polyurethane polyol prepolymer, polyol, and polyurethane polyisocyanate prepolymer must be in the ranges of about 1,000-10,000, about 150-6,000, and about 500-10,000, respectively. Preferably, the molecular weights thereof can be selected in the ranges of about 1, 400-6, 000, about 300-3, 000, and about 1,000-6,000, respectively.

The gel of the invention for an ultrasonic phantom is one constituted of the segmented polyurethane gel described above and an organic gel-swelling medium incorporated therein. This organic gel-swelling medium serves to extend the AO segments and polymer chains derived from the polyol ingredient and polyisocyanate ingredient to increase the distances between the AO segments and between the polymer chains. The gel-swelling medium thus functions to enhance ultrasonic characteristics such as ultrasonic attenuation rate and ultrasonic propagation velocity. This organic gel-swelling medium must be a nonvolatile one which does not participate in a urethane-forming reaction. It is preferred to use an organic gel-swelling medium which shows reduced absorption of ultrasonic vibration energy, has an ultrasonic propagation velocity close to that of water, and has a low attenuation rate and a satisfactory affinity for the segmented polyurethane gel (in particular, for the AO segments). Examples of such an organic gel-swelling medium include propylene carbonate, ethylene carbonate, N-methyl-2-pyrrolidone, diethylene glycol dimethyl ether, diethylene glycol diethyl ether, triethylene glycol dimethyl ether, triethylene glycol diethyl ether, tetraethylene glycol dimethyl ether, tetraethylene glycol diethyl ether, diethylene glycol dipropyl ether, diethylene glycol dibutyl ether, polypropylene glycol dimethyl ether, and polypropylene glycol diethyl ether. These compounds may be used alone or as a mixture of any two or more thereof. The polypropylene glycol dimethyl ether and polypropylene glycol diethyl ether to be used each preferably are one having a weight-average molecular weight (Mw) of 200-500.

Even when the organic gel-swellingmedium is incorporated during the formation of the segmented polyurethane gel, there is no fear that the urethane-forming reaction may be inhibited or the gel may be modified by the swelling medium, because the swelling medium does not particulate in the urethane-forming reaction. Furthermore, since the swelling medium is an organic one, it is free from germ multiplication, alteration, putrefaction, etc. In addition, since this gel-swelling medium is nonvolatile, the content thereof in the polyurethane gel does not change with time. The AO segments and polymer chains can hence be kept in a stretched state, and the gel-swelling medium itself less absorbs ultrasonic vibration energy. Consequently, the gel of the invention can continuously exhibit satisfactory ultrasonic characteristics over long. Moreover, this gel-swelling medium has a satisfactory affinity for the segmented polyurethane gel. Because of this, even when the gel-swelling medium is incorporated by causing the segmented polyurethane gel to absorb the medium, the gel-swelling medium can be rapidly absorbed in a necessary amount.

The content of the organic gel-swelling medium in the segmented polyurethane gel is preferably 20-80% by weight. In case where the content thereof is lower than 20% by weight, it is difficult to sufficiently extend the AO segments and polymer chains to increase the distances between the AO segments and between the polymer chains. It is therefore difficult to impart satisfactory ultrasonic characteristics. On the other hand, in case where the content of the organic gel-swelling medium exceeds 80% by weight, the segmented polyurethane gel comes to have reduced strength and becomes brittle. There is hence a possibility that this gel might break readily.

For incorporating the organic gel-swelling medium into the segmented polyurethane gel, use may be made of: a method in which the organic gel-swelling medium is infiltrated into and absorbed in the segmented polyurethane gel later; or a method in which the organic gel-swelling medium is mixed with either of the polyol ingredient and the polyisocyanate ingredient in forming the segmented polyurethane gel and the ingredients are reacted.

An attenuation rate regulator is suitably incorporated into this segmented polyurethane gel in order to make the ultrasonic attenuation rate of the gel close to that of target internal-organ tissues of the living body. The attenuation rate regulator may be: crosslinked or uncrosslinked beads made of any one member selected from or a mixture of any two or more members selected from the group consisting of poly (methyl methacrylate), polystyrene, polyethylene, and acrylics; or beads made of any one member selected from or a mixture of any two or more members selected from the group consisting of graphite, glass, and solid paraffin wax.
Those attenuation rate regulators each preferably have an average particle diameter of 1-100 µm. They may be used alone or as a mixture of any two or more thereof. The beads made of polymers may be expanded ones as well as unexpanded (solid) ones.

The gel for an ultrasonic phantom described above can be produced, for example, by the following processes.

In a first process, the polyol ingredient and the polyisocyanate ingredient are first mixed together in such a proportion as to result in the OH/NCO value shown above. An appropriate amount of a catalyst (e.g., dibutyltin dilaurate or a tertiary amine such as a trialkylamine or triethylenediamine) is further mixed therewith according to need. Thus, a material liquid is prepared. This material liquid is injected into a mold and reacted at ordinary or an elevated temperature to thereby produce a segmentedpolyurethane gel. Subsequently, the organic gel-swelling medium which is liquid is infiltrated and incorporated into the segmented polyurethane gel to produce a gel for an ultrasonic phantom.

In a second process, the organic gel-swelling medium is first mixed with either of the polyol ingredient and polyisocyanate ingredient. The two ingredients and an appropriate amount of a catalyst are mixed together so as to result in the OH/NCO value shown above to prepare a material liquid. Subsequently, this material liquid is injected into a mold and reacted at ordinary or an elevated temperature. Thus, a gel for an ultrasonic phantom is produced which is constituted of a segmented polyurethane gel containing the organic gel-swelling medium.

In either of those processes, when an attenuation rate regulator is to be incorporated, it is preferred to incorporate the attenuation rate regulator into either of the polyol ingredient and polyisocyanate ingredient to prepare a material liquid.

The gel for an ultrasonic phantom obtained by either of those processes is one in which the AO segments are mostly or wholly liquid at ordinary temperature and the AO segments and polymer chains derived from the polyol ingredient and polyisocyanate ingredient are kept in an extended and stretched state by the nonvolatile organic gel-swelling medium contained in the gel. Because of this, the gel is less apt to absorb ultrasonic vibration energy and has a lower ultrasonic attenuation rate than internal-organ tissues of the living body. This attenuation rate can hence be made close to the ultrasonic attenuation rate of target internal-organ tissues of the living body by adding an attenuation rate regulator. This gel for a phantom has an ultrasonic propagation velocity slightly lower than that of internal-organ tissues of the living body. However, this propagation velocity is within a range in which this velocity can be set so as to be close to the ultrasonic propagation velocity of the internal-organ tissues of the living body by regulating an ultrasonic diagnosis apparatus or the like. The ultrasonic propagation velocity of the gel hence poses no problem in practical use.

Furthermore, since the organic gel-swelling medium contained in the segmented polyurethane gel is one not participating in a urethane-forming reaction, there is no fear that the organic gel-swelling medium may inhibit the urethane-forming reaction or alter the gel even when it is mixed during gel formation. Since this gel-swelling medium is an organic one, the gel is free from germ multiplication, alteration, putrefaction, etc. Moreover, since this organic gel-swelling medium is nonvolatile and the content thereof in the polyurethane gel does not change (decrease) with time, it can keep the AO segments and polymer chains in an extended and stretched state. The gel-swelling medium itself less absorbs ultrasonic vibration energy. Consequently, the gel can continuously exhibit satisfactory ultrasonic characteristics over long. In addition, since this gel-swellingmediumhas a satisfactory affinity for the segmented polyurethane gel, the gel-swellingmediumcanbe rapidly absorbed in the segmented polyurethane gel in a necessary amount when incorporated into the gel. The gel-swelling medium can be prevented from oozing out of the segmented polyurethane gel with the lapse of time.

Examples as more specific embodiments of the invention and Comparative Example are given below.

### [EXAMPLE 1]

The polyol and the polyisocyanate each having the structure, molecular weight, and alkylene oxide (AO) segment corresponding to the structural-formula number shown in Table 1 below were prepared in the respective amounts in terms of parts shown in Table 1. Propylene carbonate as an organic gel-swelling medium and dibutyltin laurate as a catalyst were mixed with the polyol in the respective amounts in parts shown in Table 1. Thereafter, the resultant liquid mixture was mixed with the polyisocyanate to prepare a material liquid having an OH/NCO of 2.7.
This material liquid was injected into a mold and reacted at 50°C for 8 hours. Thus, a segmented polyurethane gel for an ultrasonic phantom was obtained which had EO/PO random copolymer segments having an EO/PO molar ratio of 1:1 and contained propylene carbonate as an organic gel-swelling medium in an amount of 50% by weight.

The gel for an ultrasonic phantom obtained was examined for density, and further examined for ultrasonic attenuation rate (attenuation coefficient) and ultrasonic propagation velocity with a sing-around sound velocity meter [manufactured by Ultrasonic Engineering Co., Ltd.] at 20°C and 3.5 MHz. As a result, the density, ultrasonic attenuation coefficient, and ultrasonic propagation velocity thereof were found to be 1.13 (10⁻³kg/m³), 0.13 (dB/cmMH), and 1,468 (m/s), respectively, as shown in Table 2 given later. The following were found from these results. The ultrasonic attenuation coefficient of the gel of the invention for an ultrasonic phantom was lower than the ultrasonic attenuation coefficient of human internal-organ tissues (0.5±0.05 dB/cmMH), and can be made close to the ultrasonic attenuation coefficient of the human internal-organ tissues by adding an attenuation rate regulator. The ultrasonic propagation velocity thereof also was lower than the ultrasonic propagation velocity of human internal-organ tissues (1,540±15 m/s), but is within a range in which the velocity can be set so as to be close to the ultrasonic propagation velocity of the human internal-organ tissues by regulating an ultrasonic diagnosis apparatus or the like.

After 8 months, the gel for an ultrasonic phantom was further examined for ultrasonic attenuation coefficient and ultrasonic propagation velocity. As a result, the ultrasonic attenuation coefficient and ultrasonic propagation velocity thereof were found to be 0.14 (dB/cmMH) and 1,472 (m/s), respectively, as shown in Table 2 given later. The gel showed satisfactory ultrasonic characteristics substantially equal to those measured immediately after production. This gel was completely free from germ multiplication, gel alteration, etc.

**[Table 1]**

| Polyol | Structural formula (D) Random copolymer having molecular weight, 2,000 | Parts |
|---|---|---|
| | (AO)=(CH₂CH₂O)ₘ(CH(CH₃)CH₂O), R=H m:n=1:1 | 135 |
| Polyisocyanate | Structural formula (G) Random copolymer having molecular weight, 3,000 | |
| | (AO)=(CH₂CH₂O)ₘ(CH(CH₃)CH₂O)ₙ R=(CH₂)₆ l=1 m:n=1:1 | 50 |
| Gel-swelling medium | propylene carbonate | 185 |
| Catalyst | dibutyltin dilaurate | 0.02 |

| | | |
|---|---|---|
| (OH/NCO = 2.7) | | |

### [EXAMPLES 2 TO 5]

The same procedure as in Example 1 was conducted, except that the amount in parts of the propylene carbonate as an organic gel-swelling medium was changed to each of 61.7 parts by weight, 99.6 parts by weight, 431.7 parts by weight, and 1,048 parts by weight. Thus, an ultrasonic-phantom gel containing propylene carbonate in an amount of 25% by weight (Example 2), an ultrasonic-phantom gel containing 35% by weight propylene carbonate (Example 3), an ultrasonic-phantom gel containing 70% by weight propylene carbonate (Example 4), and an ultrasonic-phantom gel containing 85% by weight propylene carbonate (Example 5) were produced.

Each gel for an ultrasonic phantom was examined for density, ultrasonic attenuation coefficient, and ultrasonic propagation velocity immediately after production and further examined for ultrasonic attenuation coefficient and ultrasonic propagation velocity after 8 months, in the same manners as in Example 1. The results obtained are shown in Table 2 below.

### [COMPARATIVE EXAMPLE 1]

The same procedure as in Example 1 was conducted, except that the organic gel-swelling medium was not mixed in preparing a material liquid. Thus, a segmented polyurethane gel was obtained which had EO/PO random copolymer segments having an EO/PO molar ratio of 1:1 and contained no organic gel-swelling medium.

This segmentedpolyurethane gel was examined for density, ultrasonic attenuation coefficient, and ultrasonic propagation velocity immediately after production and further examined for ultrasonic attenuation coefficient and ultrasonic propagation velocity after 8 months, in the same manners as in Example 1. The results obtained are shown in Table 2 below.

**[Table 2]**

| | | Example 1 | Example 2 | Example 3 | Example 4 | Example 5 | Comparative Example 1 |
|---|---|---|---|---|---|---|---|
| Propylene carbonate (wt%) | | 50 | 25 | 35 | 70 | 85 | - |
| Density (10⁻³ kg/m³) | | 1.13 | 1.08 | 1.10 | 1.16 | 1.18 | 1.04 |
| Ultrasonic propagation velocity (m/s) | Immediately after production | 1.468 | 1.493 | 1.484 | 1.461 | 1.443 | 1.506 |
| | After 8 months | 1.472 | 1.499 | 1.488 | 1.460 | 1.444 | 1.507 |
| Ultrasonic attenuation coefficient (dB/cm MH) | Immediately after production | 0.13 | 0.88 | 0.39 | 0.17 | 0.69 | 1.15 |
| | After 8 months | 0.14 | 0.88 | 0.40 | 0.18 | 0.67 | 1.14 |

The following can be seen from Table 2. The segmented polyurethane gels for phantom use of Examples 1 to 5, which contain propylene carbonate as an organic gel-swelling medium, and the segmented polyurethane gel of Comparative Example 1, which contains no organic gel-swelling medium, have respective ultrasonic propagation velocities in the range of 1,443-1,506 m/s immediately after production. Although these ultrasonic propagation velocities are lower than the ultrasonic propagation velocities of human internal-organ tissues (1, 540±15 m/s), they are within a range in which the velocities can be set so as to be close to the ultrasonic propagation velocities of the human internal-organ tissues by regulating an ultrasonic diagnosis apparatus or the like.

On the other hand, with respect to ultrasonic attenuation coefficient, the segmented polyurethane gel of Comparative Example 1, which contains no organic gel-swelling medium, has an ultrasonic attenuation coefficient of 1.15 (dB/cmMH), which is far higher than the ultrasonic attenuation coefficients of human internal-organ tissues (0.5±0.05 dB/cmMH). It can be seen that the gel of Comparative Example 1 is unsuitable for an ultrasonic phantom. In contrast, the segmented polyurethane gels of Examples 3, 1, and 4, which contain propylene carbonate as an organic gel-swelling medium in amounts of 35% by weight, 50% by weight, and 70% by weight, respectively, have ultrasonic attenuation coefficients of 0.13-0.39 (dB/cmMH). These values are lower than the ultrasonic attenuation coefficients of human internal-organ tissues, and can be made close to the ultrasonic attenuation coefficients of human internal-organ tissues by the addition of an attenuation rate regulator. It can hence be seen that the gels of those Examples are suitable for a phantom. On the other hand, the segmented polyurethane gels of Examples 2 and 5, which contain the organic gel-swelling medium in amounts of 25% by weight and 85% by weight, respectively, have ultrasonic attenuation coefficients higher than those of human internal-organ tissues. However, these values are not so high as compared with that of the gel of Comparative Example 1, and are relatively close to the ultrasonic attenuation coefficients of the human internal-organ tissues. It can be seen that the gels of Examples 2 and 5 are usable as a phantom.

It can be seen from those results of examination of the gels of Examples 1 to 5 that for obtaining a gel for phantom use which hassatisfactory ultrasonic characteristics including an ultrasonic propagation velocity close to the ultrasonic propagation velocities of human internal-organ tissues and an ultrasonic attenuation coefficient lower than the ultrasonic attenuation coefficients of human internal-organ tissues, it is preferred to incorporate the organic gel-swelling medium in an amount of 30-80% by weight.

It can be further seen that the segmented polyurethane gels for phantom use of Examples 1 to 5 do not substantially change in ultrasonic propagation velocity and ultrasonic attenuation coefficient through 8 months after production because the organic gel-swelling medium contained therein is nonvolatile, and hence continuously retain satisfactory ultrasonic characteristics. It can be seen that the gel of Comparative Example 1 also does not substantially change in ultrasonic propagation velocity and ultrasonic attenuation coefficient through 8 months after production because it is a one-component segmented polyurethane gel containing no water.

### [EXAMPLE 6]

The polyol and the polyisocyanate each having the structure, molecular weight, and alkylene oxide (AO) segment corresponding to the structural-formula number shown in Table 3 below were prepared in the respective amounts in terms of parts shown in Table 3. Propylene carbonate as an organic gel-swelling medium and dibutyltin laurate as a catalyst were mixed with the polyol in the respective amounts in parts shown in Table 3. Thereafter, the resultant liquid mixture was mixed with the polyisocyanate to prepare a material liquid having an OH/NCO of 4.0.
This material liquid was injected into a mold and reacted at 50°C for 8 hours. Thus, a segmented polyurethane gel for an ultrasonic phantom was obtained which had EO/PO random copolymer segments having an EO/PO molar ratio of 1:1 and contained propylene carbonate as an organic gel-swelling medium in an amount of 44% by weight.

This gel for an ultrasonic phantom was examined for density, ultrasonic attenuation coefficient, and ultrasonic propagation velocity immediately after production in the same manners as in Example 1. As a result, the density, ultrasonic attenuation coefficient, and ultrasonic propagation velocity thereof were found to be 1.12 (10⁻³kg/m³), 0.66 (dB/cmMH), and 1,412 (m/s), respectively.
This gel for an ultrasonic phantom was poor in shape retentivity and had flowability, because the material liquid had an OH/NCO of 4.0; this value is higher than the preferred upper limit value of 3.5 for OH/NCO.

**[Table 3]**

| Polyol | Structural formula (D) Random copolymer having molecular weight, 2,000 | Parts |
|---|---|---|
| | (AO)=(CH₂CH₂O)ₘ(CH(CH₃)CH₂O)ₙ R=H m:n=1:1 | 200 |
| Polyisocyanate | Structural formula (G) Random copolymer having molecular weight, 3,000 | |
| | (AO)=(CH₂CH₂O)ₘ(CH(CH₃)CH₂O)ₙ R=(CH₂)₆ 1=1 m:n=1:1 | 50 |
| Gel-swelling medium | propylene carbonate | 200 |
| Catalyst | dibutyltin dilaurate | 0.02 |

| | | |
|---|---|---|
| (OH/NCO = 4.0) | | |

### [EXAMPLE 7]

The polyols I and II and the polyisocyanate each having the structure, molecular weight, and alkylene oxide (AO) segment corresponding to the structural-formula number shown in Table 4 below were prepared in the respective amounts in terms of parts shown in Table 4. Propylene carbonate as an organic gel-swelling medium and dibutyltin laurate as a catalyst were mixed with the polyols in the respective amounts in parts shown in Table 4. Thereafter, the resultant liquid mixture was mixed with the polyisocyanate to prepare a material liquid having an OH/NCO of 2.8. This material liquid was reacted under the same conditions as in Example 1. Thus, a segmented polyurethane gel for an ultrasonic phantom was obtained which had EO/PO block copolymer segments and contained propylene carbonate as an organic gel-swelling medium in an amount of 50% by weight.

This gel for an ultrasonic phantom was examined for density, ultrasonic attenuation coefficient, and ultrasonic propagation velocity immediately after production in the same manners as in Example 1 . As a result, the density, ultrasonic attenuation coefficient, and ultrasonic propagation velocity thereof were found to be 1.13 (10⁻³kg/m³), 0.29 (dB/cmMH), and 1,462 (m/s), respectively. At 8 months after the production, the gel was further examined for density, ultrasonic attenuation coefficient, and ultrasonic propagation velocity. As a result, almost the same values as those immediately after production were obtained.

**[Table 4]**

| Polyol I | Structural formula (B) Block copolymer having molecular weight, 3,000 | Parts |
|---|---|---|
| | (AO)=(CH(CH₃)CH₂O)ₘ(CH₂CH₂O)ₙ l=1 m:n=1:1 | 70 |
| Polyol II | Structural formula (D) Block copolymer having molecular weight, 1,670 | |
| | (AO)=(CH₂CH₂O)ₘ(CH(CH₃)CH₂O)ₚ(CH₂CH₂O)ₙ R=H m:p:n=1:0.5:1 | 58.5 |
| Polyisocyanate | Structural formula (G) Random copolymer having molecular weight, 3,000 | |
| | (AO)=(CH₂CH₂O)ₘ(CH(CH₃)CH₂O)ₙ R=(CH₂)₆ 1=1 m:n=1:1 | 50 |
| Gel-swelling medium | propylene carbonate | 178.5 |
| Catalyst | dibutyltin dilaurate | 0.02 |

| | | |
|---|---|---|
| (OH/NCO = 2.8) | | |

### [EXAMPLE 8]

The polyol and the polyisocyanate each having the structure, molecular weight, and alkylene oxide (AO) segment corresponding to the structural-formula number shown in Table 5 below were prepared in the respective amounts in terms of parts shown in Table 5. Tetraethylene glycol dimethyl ether as an organic gel-swelling medium and dibutyltin laurate as a catalyst were mixed with the polyol in the respective amounts in parts shown in Table 5. Thereafter, the resultant liquid mixture was mixed with the polyisocyanate to prepare a material liquid having an OH/NCO of 2.4.
This material liquid was injected into a mold and reacted under the same conditions as in Example 1. Thus, a segmented polyurethane gel for an ultrasonic phantom was obtained which had EO/PO random copolymer segments having an EO/PO molar ratio of 1:1 and contained tetraethylene glycol dimethyl ether as an organic gel-swelling medium in an amount of 50% by weight.

This gel for an ultrasonic phantom was examined for density, ultrasonic attenuation coefficient, and ultrasonic propagation velocity immediately after production in the same manners as in Example 1. As a result, the density, ultrasonic attenuation coefficient, and ultrasonic propagation velocity thereof were found to be 1.03 (10⁻³ kg/m³), 0.31 (dB/cmMH), and 1,465 (m/s), respectively. At 8 months after the production, the gel was further examined for density, ultrasonic attenuation coefficient, and ultrasonic propagation velocity. As a result, almost the same values as those immediately after production were obtained.

**[Table 5]**

| Polyol | Structural formula (D) Random copolymer having molecular weight, 2,000 | Parts |
|---|---|---|
| | (AO)=(CH₂CH₂O)ₘ(CH(CH₃)CH₂O)ₙ R=H m:n=1:1 | 120 |
| Polyisocyanate | Structural formula (G) Random copolymer having molecular weight, 3,000 | |
| | (AO)=(CH₂CH₂O)ₘ(CH(CH₃)CH₂O)ₙ R=(CH₂)₆ l=1 m:n=1:1 | 50 |
| Gel-swelling medium | tetraethylene glycol dimethyl ether | 120 |
| Catalyst | dibutyltin dilaurate | 0.01 |

| | | |
|---|---|---|
| (OH/NCO = 2.4) | | |

While the invention has been described in detail and with reference to specific embodiments thereof, it will be apparent to one skilled in the art that various changes and modifications can be made therein without departing from the spirit and scope thereof. This application is based on a Japanese patent application filed on November 29, 2005 (Application No. 2005-343909), the entire contents thereof being herein incorporated by reference.

### INDUSTRIAL APPLICABILITY

According to the invention, a gel for an ultrasonic phantom can be provided which has sufficient ultrasonic characteristics, does not fluctuate in ultrasonic characteristics over long, and is free fromgermmultiplication, alteration, putrefaction, etc.

## Claims

1. A gel for an ultrasonic phantom, which comprises:
a segmented polyurethane gel having alkylene oxide segments which are mostly or wholly liquid at ordinary temperature;
and a nonvolatile organic gel-swelling medium not participating in a urethane-forming reaction, wherein the medium is contained in the segmented polyurethane gel.

2. The gel for an ultrasonic phantom according to claim 1, wherein the alkylene oxide segments each is a copolymer of ethylene oxide and propylene oxide.

3. The gel for an ultrasonic phantom according to claim 2, wherein the molar ratio of ethylene oxide to propylene oxide in the copolymer is from 20:80 to 80:20.

4. The gel for an ultrasonic phantom according to claim 1 or 2, wherein the organic gel-swelling medium is contained in the segmented polyurethane gel in an amount of 20-80% by weight.

5. The gel for an ultrasonic phantom according to any one of claim 1 to 4, wherein the organic gel-swelling medium comprises any one member selected from or a mixture of any two or more members selected from propylene carbonate, ethylene carbonate, N-methyl-2-pyrrolidone, diethylene glycol dimethyl ether, diethylene glycol diethyl ether, triethylene glycol dimethyl ether, triethylene glycol diethyl ether, tetraethylene glycol dimethyl ether, tetraethylene glycol diethyl ether, diethylene glycol dipropyl ether, diethylene glycol dibutyl ether, polypropylene glycol dimethyl ether, and polypropylene glycol diethyl ether.

6. The gel for an ultrasonic phantom according to any one of claim 1 to 4, wherein the segmented polyurethane gel contains an attenuation rate regulator for regulating ultrasonic attenuation rate.

7. The gel for an ultrasonic phantom according to claim 6, wherein the attenuation rate regulator comprises a crosslinked or uncross linked bead made of any one member selected from or a mixture of any two or more members selected from the group consisting of poly (methyl methacrylate), polystyrene, polyethylene, and acrylics or comprises bead made of any one member selected from or a mixture of any two or more members selected from the group consisting of graphite, glass, and solid paraffin wax.

8. The gel for an ultrasonic phantom according to claim 7, wherein the attenuation rate regulator has an average particle diameter of 1-100 µm.
